(19) Europäisches Patentamt
European Patent Office
Office européen des brevets

(11) **EP 3 438 646 A1**

(12) **EUROPEAN PATENT APPLICATION**
published in accordance with Art. 153(4) EPC

(43) Date of publication:
**06.02.2019 Bulletin 2019/06**

(51) Int Cl.:
***G01N 21/17*** *(2006.01)*

(21) Application number: **17773534.7**

(22) Date of filing: **20.01.2017**

(86) International application number:
**PCT/JP2017/001858**

(87) International publication number:
**WO 2017/168984 (05.10.2017 Gazette 2017/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **28.03.2016 JP 2016063744**

(71) Applicant: **Sony Corporation
Tokyo 108-0075 (JP)**

(72) Inventors:
• **NAKAO, Isamu
Tokyo 108-0075 (JP)**
• **KISHIMOTO, Takuya
Tokyo 108-0075 (JP)**
• **KISHII, Noriyuki
Tokyo 108-0075 (JP)**

(74) Representative: **D Young & Co LLP
120 Holborn
London EC1N 2DY (GB)**

(54) **IMAGING DEVICE, IMAGING METHOD**

(57) A fluid located at a certain depth in an imaging object can be imaged with high sensitivity and accuracy. As a result, an imaging technique by which information regarding the fluid can be accurately obtained is provided. An imaging apparatus includes: a light irradiation unit that irradiates a plurality of positions of an imaging object with coherent light as spots; a light detection unit that detects light, the light being emitted from the light irradiation unit and propagated in the imaging object; and an imaging unit that captures a speckle image obtained from scattered light of the imaging object, the scattered light being detected by the light detection unit.

FIG.2

## Description

Technical Field

The present technology relates to an imaging apparatus and an imaging method. More particularly, the present technology relates to an imaging apparatus and an imaging method that use speckles generated by irradiating an imaging object with light.

Background Art

Conventionally, in order to grasp the shape, structure, and the like of a biological sample such as a blood vessel and a cell, an imaging apparatus and an imaging method using an optical method have been developed.
As those imaging apparatus and the like, there are known an imaging apparatus and the like disclosed by Patent Literature 1. In the imaging apparatus and the like disclosed by Patent Literature 1, an optical sensor includes an irradiation system including a plurality of light source modules (light irradiators) that irradiate an object under test (pseudo-living body) with light and a detection system that detects light emitted from the irradiation system and propagated in the object under test. Further, each of the plurality of light source modules irradiates the same position of the object under test with a plurality of non-parallel light beams. In this case, the resolution can be improved without impairing the attachment performance to the object under test.
In such an imaging technique using an optical method in the case of using a flow path such as a blood vessel as an imaging object, there is a concern that occurrence of various types of noise may cause detection accuracy to deteriorate. As a kind of noise, speckles are well-known. The speckles refers to a phenomenon that a spot-like swaying pattern appears on an irradiated surface in a manner that depends an uneven shape of the irradiated surface. In recent years, techniques have also been developed with respect to a method of imaging a flow path such as a blood vessel by using speckles which are a kind of noise.
By the way, speckles are a random interference/diffraction pattern due to scattering or the like in an optical path. In addition, the magnitude of speckles is represented by an index called speckle contrast which is a value obtained by dividing the standard deviation of the intensity distribution by the average of the intensity distribution. When the imaging object irradiated with coherent light is observed by using an imaging optical system, the speckles caused by scattering of the imaging object are observed on the image plane. When the imaging object moves or changes in shape, a random speckle pattern corresponding to the movement or change is observed.
When a light scattering fluid such as blood is observed, the speckle pattern changes at every moment according to the change in fine shape caused by the flow. At that time, when an imaging element is arranged on the image plane and the fluid is imaged with an exposure time sufficiently longer than the change of the speckle pattern, the speckle contrast of a portion in which the blood is flowing, that is, a portion of the blood vessel is reduced in time average. Angiography can be performed by using such a change in speckle contrast.
As an imaging technique using the speckles as described above, there is known a technique disclosed by Non-Patent Literature 1 (see Non-Patent Literature 1) .
In this technique disclosed by Non-Patent Literature 1, an imaging object containing a fluid is irradiated with laser light at a single point and blood-flow information provided due to speckles from information of an image of the surrounding object is obtained.

Citation List

Patent Literature

Patent Literature 1: Japanese Patent Application Laid-open No. 2015-092151

Non-Patent Literature

Non-Patent Literature 1: Renzhe Bi et al., "OPTICS LETTERS Vol. 38, No. 9" May 1, 2013

Disclosure of Invention

Technical Problem

However, the imaging technique described in Patent Literature 1 is limitedly used for observing a vicinity of a surface of an imaging object, and thus, it is impossible to detect a blood vessel, blood, and the like located at a certain depth in the imaging object.

Moreover, in the imaging technique described in Non-Patent Literature 1, the single point of the imaging object is irradiated with laser light. Therefore, when imaging is to be performed, it is necessary to scan the light source that emits laser light and the detection system for detecting the laser light. Thus, there is a problem that it is difficult to display speckles in real time.

In view of this, it is a main object of the present technology to provide an imaging technique by which even if a fluid contained in an imaging object is located at a predetermined depth or more in the imaging object, that fluid can be suitably imaged and further, speckles can be displayed in real time.

Solution to Problem

The present technology provides an imaging apparatus including: a light irradiation unit that irradiates a plurality of positions of an imaging object with coherent light as spots; a light detection unit that detects light, the light being emitted from the light irradiation unit and propagated in the imaging object; and an imaging unit that captures a speckle image obtained from scattered light of the imaging object, the scattered light being detected by the light detection unit.

In the imaging apparatus according to the present technology, the light irradiation unit may be configured to irradiate the plurality of positions of the imaging object with light as spots in such a manner that the spots are arranged at predetermined intervals on the imaging object.

Moreover, in the imaging apparatus according to the present technology, an optical path of the light propagated in the imaging object may be represented by Expression 1 as follows.

[Expression 1]

$$Z(x) \approx \sqrt{\frac{1}{8}\left\{\sqrt{\left[x^2 + (r-x)^2\right]^2 + 32x^2(r-x)^2} - x^2 - (r-x)^2\right\}}$$

$$r\sqrt{3\mu_a\mu_s(1-g)} << 1$$

In addition, the imaging apparatus according to the present technology may further include an image processing unit that determines whether or not light is detected by the light detection unit on a basis of imaging information obtained by the imaging unit.

Moreover, the imaging apparatus according to the present technology may further include a distance calculating unit that calculates, on a basis of Expression 1, a distance between a spot irradiation position of the light irradiation unit and a detection position of the light propagated in the imaging object.

The imaging apparatus according to the present technology may further include an irradiation switching unit that switches an irradiation method of the light irradiation unit from spot irradiation to uniform irradiation.

Moreover, the imaging apparatus according to the present technology may further include a combined-image generating unit that generates a combined image on a basis of a plurality of pieces of light information detected by the light detection unit.

The present technology also provides an imaging method including: a light irradiation step of irradiating a plurality of positions of an imaging object with coherent light as spots; a light detecting step of detecting light, the light being emitted as spots and propagated in the imaging object; and an imaging step of capturing a speckle image obtained from scattered light of the imaging object, the scattered light being detected in the light detecting step.

In the imaging method according to the present technology, the light irradiation step may include emitting the coherent light to the plurality of positions of the imaging object in such a manner that light beams of the coherent light are arranged at predetermined intervals on the imaging object.

Moreover, in the imaging method according to the present technology, the light propagated in the imaging object may be represented by Expression 2 as follows.

[Expression 2]

$$Z(x) \approx \sqrt{\frac{1}{8}\left\{\sqrt{\left[x^2+(r-x)^2\right]^2+32x^2(r-x)^2}-x^2-(r-x)^2\right\}}$$

$$r\sqrt{3\mu_a\mu_s(1-g)} << 1$$

In addition, the imaging method according to the present technology may further include an image processing step of determining whether or not light is detected in the light detecting step on a basis of imaging information obtained in the imaging step.

Moreover, the imaging method according to the present technology may further include a distance calculating step of calculating, on a basis of Expression 2, a distance between a spot irradiation position in the light irradiation step and a light detection position in the light detecting step.

In addition, the imaging method according to the present technology may further include an irradiation switching step of switching an irradiation method for the coherent light from spot irradiation to uniform irradiation.

Moreover, the imaging method according to the present technology may further include a combined-image generating step of generating a combined image on a basis of a plurality of pieces of light information detected in the light detecting step.

Advantageous Effects of Invention

In accordance with the present technology, a fluid located at a certain depth in an imaging object can be imaged with high sensitivity and accuracy. As a result, information regarding the fluid can be accurately obtained.

It should be noted that the effects described here are not necessarily limitative and any effect described in the present technology may be provided.

Brief Description of Drawings

[Fig. 1] Fig. 1 is a schematic conceptual diagram schematically showing a concept of a first embodiment of an imaging apparatus according to the present technology.

[Fig. 2] A block diagram showing details of the imaging apparatus shown in Fig. 1.

[Fig. 3] A schematic conceptual diagram schematically showing an optical path of coherent light in the imaging apparatus of the first embodiment.

[Fig. 4] A diagram for describing suitable optical paths of coherent light in the imaging apparatus according to the present technology.

[Fig. 5] An overview diagram showing an example of a method of generating a pattern of spot irradiation by a light irradiation unit according to the present technology.

[Fig. 6] An overview diagram showing an example of the method of generating the pattern of spot irradiation by the light irradiation unit according to the present technology.

[Fig. 7] An overview diagram showing an example of the method of generating the pattern of spot irradiation by the light irradiation unit according to the present technology.

[Fig. 8] A block diagram showing an overview of a second embodiment of the imaging apparatus according to the present technology.

[Fig. 9] A graph substituting diagram showing a relationship between a spot irradiation position and a depth of an imaging object.

[Fig. 10] A graph substituting diagram showing a relationship between the spot irradiation position and the depth of the imaging object.

[Fig. 11] A block diagram showing an overview of a third embodiment of the imaging apparatus according to the present technology.

[Fig. 12] A block diagram showing an overview of a fourth embodiment of the imaging apparatus according to the present technology.

[Fig. 13] A flowchart showing a first embodiment of an imaging method according to the present technology.

[Fig. 14] A flowchart showing a second embodiment of the imaging method according to the present technology.
[Fig. 15] A flowchart showing a third embodiment of the imaging method according to the present technology.
[Fig. 16] A flowchart showing a fourth embodiment of the imaging method according to the present technology.
Mode(s) for Carrying Out the Invention

Suitable embodiments for implementing the present technology will be described below with reference to the drawings. Each embodiment to be described below shows an example of a representative embodiment of the present technology, so that the scope of the present technology is not to be narrowly interpreted by the embodiments. It should be noted that the description will be made in the following order.

1. Imaging Apparatus According to First Embodiment

(1) Light Irradiation Unit
(2) Light Detection Unit
(3) Speckle Imaging Unit
(4) Combined-Image Generating Unit
(5) Analysis Unit
(6) Storage Unit
(7) Display Unit
(8) Imaging Object

2. Imaging Apparatus According to Second Embodiment

(1) Irradiation Switching Unit

3. Imaging Apparatus According to Third Embodiment

(1) Image Processing Unit

4. Imaging Apparatus According to Fourth Embodiment

(1) Distance Calculating Unit

5. Imaging Method According to First Embodiment

(1) Light Irradiation Step
(2) Light Detecting Step
(3) Speckle Imaging Step
(4) Combined-Image Generating Step
(5) Analyzing Step
(6) Storing Step
(7) Displaying Step

6. Imaging Method According to Second Embodiment

(1) Depth Measuring Step
(2) Irradiation Switching Step

7. Imaging Method According to Third Embodiment (1) Image Processing Step
8. Imaging Method According to Fourth Embodiment (1) Distance Calculating Step

<1. Imaging Apparatus According to First Embodiment>

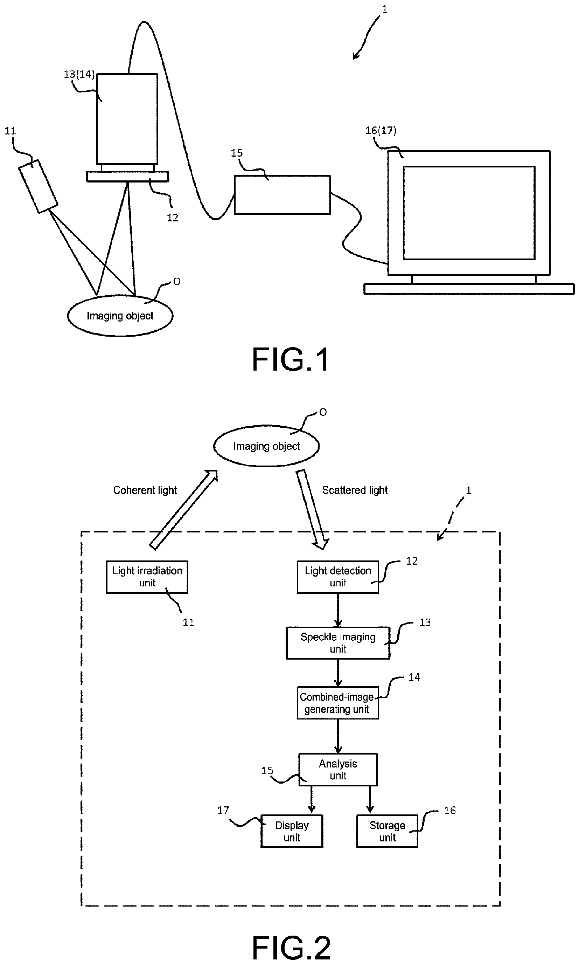

A first embodiment of an imaging apparatus according to the present technology will be described with reference to Figs. 1 to 7.
An imaging apparatus 1 shown in Figs. 1 and 2 at least includes a light irradiation unit 11, a light detection unit 12, and a speckle imaging unit 13. Moreover, the imaging apparatus 1 may further include, as necessary, a combined-image generating unit 14, an analysis unit 15, a storage unit 16, a display unit 17, and the like. Hereinafter, each of the units will be described in detail.

(1) Light Irradiation Unit

The light irradiation unit 11 irradiates an imaging object O with coherent light. That coherent light refers to light in which the phase relationship between light waves at arbitrary two points in a light flux is invariable and constant in terms of time and, thus, even in the case of dividing the light flux by an arbitrary method and, after that, providing a large optical path difference and overlaying the divided light fluxes again, perfect coherency is exhibited.

Laser light is favorable as the coherent light. As the light source 11 that emits laser light, for example, an argon ion (Ar) laser, a helium-neon (He-Ne) laser, a dye laser, a krypton (Cr) laser, a distributed feedback (DFB) or grating feedback semiconductor laser, and the like can be used.

Moreover, a wavelength number of the coherent light is not particularly limited, and can be changed as appropriate in a manner that depends on the configurations and the types of the imaging object O and/or the fluid contained in the same. For example, in a case where the fluid is blood flowing in a blood vessel, it is favorable that the wavelength number of the coherent light is within a range of 600 to 1550 nm and it is red light that provides a low light absorption rate.

In addition, the light irradiation unit 11 according to the present technology irradiates a plurality of positions of the imaging object O with coherent light as spots.

Note that when the imaging object O is irradiated with the coherent light as spots, the coherent light is propagated in the imaging object O and then is detected by the light detection unit 12.

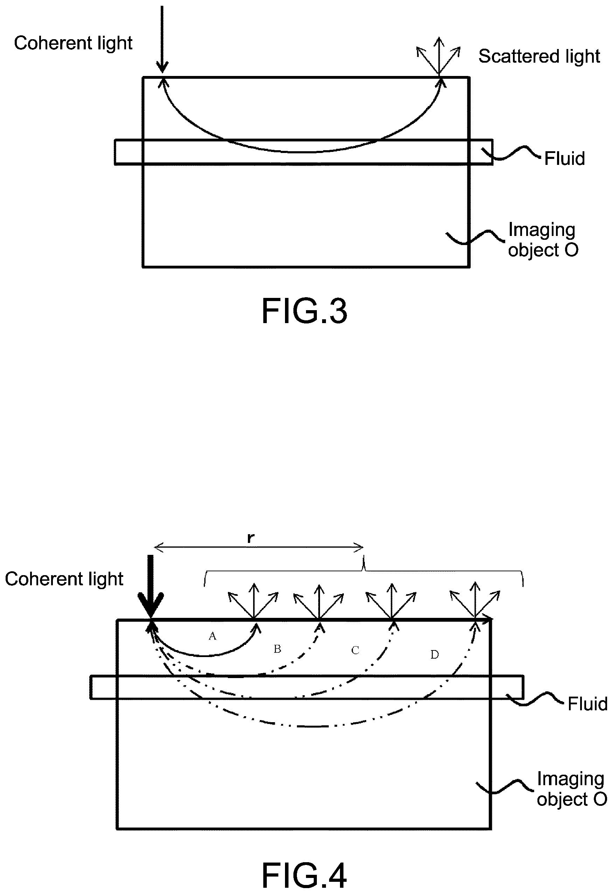

In this case, as shown in Fig. 3, the coherent light is propagated in the imaging object O in an approximately circular arc form.

That is, in a case where the coherent light is detected at a point different from the irradiation position, a most likely optical path through which it can arrive at the detection position is represented by Expression 3 as follows.

[Expression 3]

$$Z(x) \approx \sqrt{\frac{1}{8}\left\{\sqrt{\left[x^2 + (r-x)^2\right]^2 + 32x^2(r-x)^2} - x^2 - (r-x)^2\right\}}$$

$$r\sqrt{3\mu_a\mu_s(1-g)} << 1$$

In Expression 3, "r" denotes a distance from the irradiation position to the detection position, "$\mu_a$" denotes an equivalent absorption coefficient, "$\mu_s$" denotes an equivalent scattering coefficient, and G denotes an anisotropy coefficient.

Further, a depth when the coherent light enters most deeply in the optical path represented by Expression 3 is represented by Expression 4 as follows.

[Expression 4]

$$Z_{depth} = \frac{\sqrt{2}}{4}r$$

As can be seen from Expressions 3 and 4, in proportion to an increase or decrease of the distance from the irradiation position to the detection position, the depth at which the coherent light enters also increases or decreases.

It should be noted that, in a case where the coherent light is detected at a point different from the irradiation position, the coherent light is surely propagated through the optical path represented by Expression 3.

Therefore, as shown in Fig. 4, there are, for example, a case where the coherent light is not propagated in a fluid contained in the imaging object O (optical path A), a case where the coherent light is propagated in the fluid (optical path B and optical path C), and a case where the coherent light is propagated in a portion of the imaging object O, which is deeper than the fluid (optical path D).

In such a case, on the basis of the coherent light propagated through the optical path A, optical information regarding the fluid cannot be obtained as a matter of course. Moreover, on the basis of the coherent light propagated through the optical path D, there is a possibility that optical signals from portions of the imaging object O other than the fluid are detected as noise, which is not favorable.

However, the optical path of the coherent light is favorably the optical path B or C because there is a low possibility that optical signals of the portions other than the fluid are detected as noise.

In the imaging apparatus 1 according to the present technology, the plurality of positions of the imaging object O are irradiated with the coherent light as spots to be propagated through the optical path. Regarding the number of spot irradiation positions, it is only necessary to avoid a configuration in which the light irradiation unit 11 irradiates only one point of the imaging object O with light as a spot. Thus, the number of spot irradiation positions only needs to be at least two or more. Moreover, a pattern of spot irradiation when the plurality of positions of the imaging object O are irradiated with light as spots is also not particularly limited, and the spot irradiation positions adjacent to each other only need to be arranged at predetermined intervals. Regarding the term "at predetermined intervals", the predetermined intervals only need to prevent optical information based on adjacent spot irradiation at the spot irradiation positions adjacent to each other from appearing as noise for optical information for acquiring an image.

Therefore, for example, the pattern of the spot irradiation positions may be a state in which a plurality of spot irradiation positions are regularly arrayed or may be arranged in spots on the imaging object O.

Further, a method of calculating a distance between the respective spot irradiation positions is not particularly limited, and a well-known method can be employed. For example, a single spot irradiation position is first generated on the imaging object O. With this spot irradiation, a blood flow/blood vessel is detected by the optical path according to Expression 3, speckle contrast is locally calculated, and a two-dimensional speckle contrast map is generated within reach of diffused light.

In addition, a position at which a decrease of the speckle contrast becomes smallest in this map is extracted, and a distance r from a spot center to that minimum point is calculated. A value twice the distance r is used as an irradiation spot distance in actual irradiation.

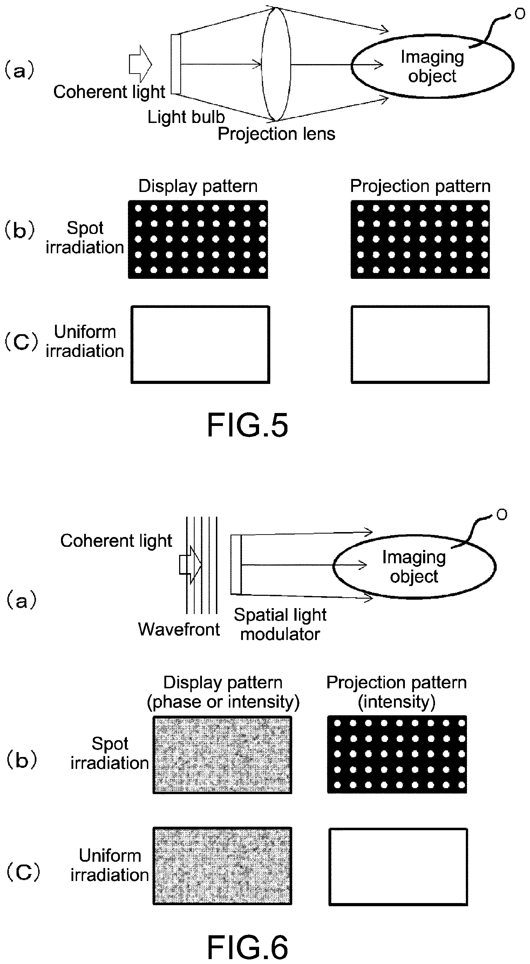

Moreover, a method of generating the pattern of the spot irradiation positions is also not particularly limited. An example of this pattern generating method will be described with reference to Figs. 5 to 7.

As the generating method, there is, for example, a method of projecting a pattern by using image formation of the lens (see Fig. 5). It should be noted that in Fig. 5, (a) is an overview diagram of the pattern generating method, (b) shows an example of generated display and projection patterns, and (c) shows an example of the pattern in a case where the imaging object O is uniformly irradiated with the coherent light in the method shown in (a).

In this method, a method in which a pattern to be projected is displayed on a transmissive display device such as a light bulb and an irradiation spot pattern is displayed on the imaging object O by using image formation of a projection lens is conceivable.

At this time, the light bulb is irradiated with the coherent light from the back side. Regarding an arrangement relationship between the light bulb and the projection lens, the projection lens and the imaging object O onto which the pattern is to be projected, an image formation relationship represented by Expression 5 as follows is kept with respect to an optical axis.

[Expression 5]

$$\frac{1}{S_0} + \frac{1}{S_1} = \frac{1}{f}$$

It should be noted that $S_0$ and $S_1$ respectively denote a distance from the light bulb to the projection lens and a distance from the projection lens to the imaging object O, and f denotes a focal distance of the projection lens.

It should be noted that although the transmissive light bulb is used in the method shown in Fig. 5, the spot pattern can also be generated by using a reflective light bulb. In this case, the coherent light is emitted from the front side of the display device.

As another example of the pattern generating method, there is a method of projecting a pattern by using holography as shown in Fig. 6. It should be noted that in Fig. 6, (a) is an overview diagram of the pattern generating method, (b) shows an example of generated display and projection patterns, and (c) shows an example of the pattern in a case where the imaging object O is uniformly irradiated with the coherent light in the method shown in (a).

Specifically, as shown in (a) of Fig. 6, a spatial light modulator is irradiated with the coherent light such as a plane wave from the back side or the front side. A diffraction pattern of a hologram, which is spatially modulated in terms of the phase or the intensity by the spatial light modulator, is thus formed in spots on the imaging object O (see right-hand side of (b) of Fig. 6).

In this case, the spot pattern having desired intensity is formed on the imaging object O. Therefore, it is favorable to calculate a hologram pattern output to the spatial light modulator by using the Fourier transform or the Fresnel transform in a manner that depends on a projection distance.

Note that in general, the hologram pattern can be mathematically determined as a complex amplitude while the spatial light modulator can modulate only either one of the phase and the intensity. Therefore, it is favorable to calculate a hologram pattern from which a desired pattern can be obtained by successive iterative calculation with the Fourier transform or the Fresnel transform while changing various parameters such that the desired pattern can be obtained.

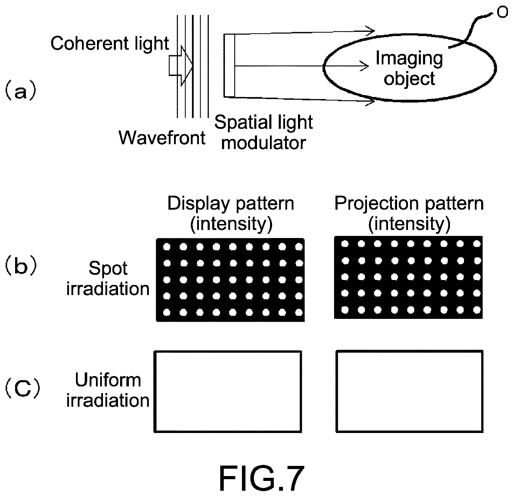

As another example of the pattern generating method, there is a method of projecting a pattern by using the Talbot effect as shown in Fig. 7. It should be noted that in Fig. 7, (a) is an overview diagram of the pattern generating method, (b) shows an example of generated display and projection patterns, and (c) shows an example of the pattern in a case where the imaging object O is uniformly irradiated with the coherent light in the method shown in (a).

Note that in accordance with Non-Patent Literature (F. Talbot, Facts relating to optical science IV, Philos. Mag. 9 (1836) 401-407., L. Rayleigh, On copying diffraction-gratings, and on some phenomenon connected therewith, Philos. Mag. 11 (1881) 196-205.), it is known that when a periodic diffraction grating is irradiated with high coherent light, an image of the diffraction grating is formed at integer n multiples of the Talbot length of Expression 6 as follows with respect to the optical axis is formed, and an image of a pattern having a cycle obtained by multiplying the cycle of the diffraction grating by $2^{-(n-1)}$ at intervals of $2^{-(n-1)}$ is formed.

[Expression 6]

$$z_T = \frac{2a^2}{\lambda}$$

In the method shown in Fig. 7, a desired pattern to be projected or a pattern having a cycle obtained by multiplying it by $2 \times n$ is generated in the spatial light modulator of the intensity modulation type and the spatial light modulator of the intensity modulation type is irradiated with coherent light by using a distance from the spatial light modulator to the imaging object O as a distance satisfying the image formation condition. An irradiation pattern having a spot array shape can be thus formed (see right-hand side of (b) of Fig. 7).

The coherent light emitted from the light irradiation unit 11 according to the present technology as described above and propagated in the imaging object O is detected by the light detection unit 12.

(2) Light Detection Unit

The imaging apparatus 1 according to the present technology includes the light detection unit 12 that detects the coherent light emitted from the light irradiation unit 11.

Specifically, the light detection unit 12 is configured to convert optical signals based on scattered light or reflected light emitted from the imaging object O due to irradiation of coherent light into electrical signals. The configuration and aspect of this light detection unit 12 are not particularly limited, and a well-known configuration can be employed. For example, a configuration including reflected-light detector that detects only reflected light emitted from the imaging object O and a scattered-light detector that detects only scattered light emitted from the imaging object O and the like are conceivable.

(3) Speckle Imaging Unit

In the speckle imaging unit 13, imaging of speckles appearing on a surface of the imaging object O is performed on the basis of scattered light obtained from the imaging object O irradiated with the coherent light of each light irradiation unit 11.

Specifically, in the imaging apparatus 1 according to the present technology, on the basis of electrical signals generated at detection positions corresponding to the respective spot irradiation positions, a plurality of speckle images due to the respective spot irradiation positions are captured.

This speckle imaging unit 13 includes, for example, an imaging optical system that forms an image of the scattered light obtained from the imaging object O, and an imaging system that receives the light of the image formed by the imaging optical system. The imaging optical system includes an imaging element such as a CCD sensor or a CMOS sensor, an imaging lens, and the like. In the CMOS sensor, a global shutter system and a rolling shutter system are known, and any of the systems can be employed in the imaging apparatus 1 according to the present technology.

The imaging method performed by the speckle imaging unit 13 is not particularly limited as long as the effect of the present technology is not impaired, and one type or two types or more of well-known imaging methods may be selected and used freely in combination. For example, there is an imaging method using the imaging element described above.

In this speckle imaging unit 13, for example, an image or the like in which a pseudo blood vessel through which pseudo blood flows is mapped on the basis of the speckles is generated. Since the speckles are a random interference/diffraction pattern as described above, when a light scattering fluid such as blood moves or changes with time, the speckles also vary with time. For this reason, it is possible to observe the boundary between the fluid and other portions.

It should be noted that, in order to more clarify the portion where speckles are generated, the speckle imaging unit 13 may have a configuration in which, for example, equalization is performed by using a plurality of speckle images to reduce irregularity of the speckle images.

Moreover, the speckle imaging unit 13 may include a filter that blocks external light so as to be capable of positively taking in the scattered light from the imaging object O.

(4) Combined-Image Generating Unit

The imaging apparatus 1 according to the present technology favorably includes the combined-image generating unit 14 that combines a plurality of speckle images generated by the speckle imaging unit 13.

A method of combining a plurality of speckle images, which is performed by the combined-image generating unit 14, is not particularly limited, and a great variety of methods are conceivable. For example, a method of generating one speckle combined image by making image information (e.g., the number of pixels, the color tone of pixels, and the like) of the respective speckle images the same, a method of simply making the respective speckle images overlap each other, and the like are conceivable.

It should be noted that the combined image generated by the combined-image generating unit 14 needs to be generated such that the state of the imaging object O can be analyzed on the basis of the generated combined image. Therefore, in the combined-image generating unit 14, for example, a method of correcting, on the basis of luminance values of each speckle image, the luminance values of the speckle combined image such that the luminance distribution in the speckle combined image becomes uniform and the like may be performed.

(5) Analysis Unit

The imaging apparatus 1 according to the present technology can include the analysis unit 15 that analyzes the state of the imaging object O on the basis of the speckle image captured by the speckle imaging unit 13 or the combined image generated by the combined-image generating unit 14.

In this analysis unit 15, for example, an intensity distribution of speckles is measured by using a luminance meter and the like on a speckle image captured by the speckle imaging unit 13.

Using a result of the measurement, speckle contrast, which is a value obtained by dividing the standard deviation of the intensity distribution by the average of the intensity distribution, is measured. By such measurement of speckle contrast, angiography can be performed by using a change in speckle contrast in a case where the imaging object O is assumed as a blood vessel being a light scattering fluid. Furthermore, since the speckles vary with time, the speed of the blood flow can also be analyzed.

It should be noted that a method of measuring the intensity distribution of the speckles or the speckle contrast is not particularly limited as long as the effect of the present technology is not impaired, and one type or two types or more of well-known measurement methods may be selected and used freely in combination.

(6) Storage Unit

The imaging apparatus 1 according to the present technology can further include the storage unit 16 that stores the speckle image captured by the speckle imaging unit 13, the combined image generated by the combined-image generating unit 14, the speckle contrast measured by the analysis unit 15, the analysis result obtained by the analysis unit 15, and the like as necessary.

This storage unit 16 is not necessarily provided in the imaging apparatus 1 according to the present technology, and, for example, an external storage device can also be connected to store the speckle image and the like.

(7) Display Unit

The imaging apparatus 1 according to the present technology can further include the display unit 17 that displays the speckle image captured by the speckle imaging unit 13, the combined image generated by the combined-image generating unit 14, the analysis result obtained by the analysis unit 15, and the like. This display unit 17 is not necessarily provided in the imaging apparatus according to the present technology, and, for example, an external monitor or the like can also be used.

(8) Imaging Object O

Although the imaging apparatus 1 according to the present technology may use various objects as the imaging objects, the image analysis apparatus can be suitably used for imaging an object containing, for example, a fluid as the imaging object. Due to the nature of the speckles, the speckles are not easily generated from the fluid. For this reason, when the object containing a fluid is imaged by using the imaging apparatus 1 according to the present technology, a boundary between the fluid and other portions, a flow speed of the fluid, and the like can be obtained.
More specifically, a biological sample may be exemplified as the imaging object O and blood may be exemplified as the fluid. For example, when the imaging apparatus 1 according to the present technology is mounted on a surgical microscope, a surgical endoscope, or the like, surgery can be performed while identifying the position of a blood vessel. Therefore, it is possible to carry out safer and highly accurate surgery, and thus, it is possible to contribute to further development of the medical technology.
In accordance with the thus configured imaging apparatus 1 according to the present technology, the coherent light is propagated through a particular optical path in the imaging object O due to the spot irradiation of the light irradiation unit 11 and the light detection unit 12 detects the coherent light propagated through the optical path.
Therefore, even if the fluid contained in the imaging object O is located at a deep position in the imaging object O, it is possible to suitably image the fluid by using the speckles and to accurately analyze the state of the fluid.
In addition, the light irradiation unit 11 irradiates the plurality of positions of the imaging object O with light and the speckle image is generated on the basis of the optical information emitted from each of the detection positions. Therefore, it is possible to display the speckles in real time, and thus to obtain information (speed and the like) regarding the fluid at the deep portion with higher sensitivity and accuracy than typical uniform irradiation.

<2. Imaging Apparatus According to Second Embodiment>

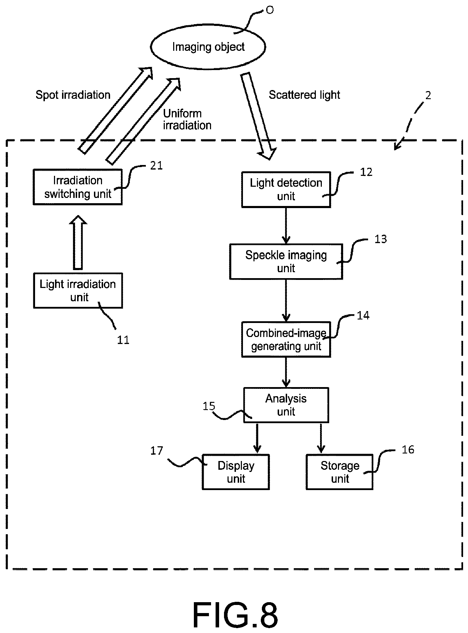

Next, a second embodiment of the imaging apparatus according to the present technology will be described with reference to Figs. 8 to 10.
An imaging apparatus 2 according to the second embodiment is different from the imaging apparatus 1 according to the first embodiment in that the imaging apparatus 2 according to the second embodiment includes an irradiation switching unit 21. On the other hand, other configurations are identical to those of the imaging apparatus 1 according to the first embodiment, and thus will be denoted by identical signs and descriptions thereof will be omitted.

(1) Irradiation Switching Unit

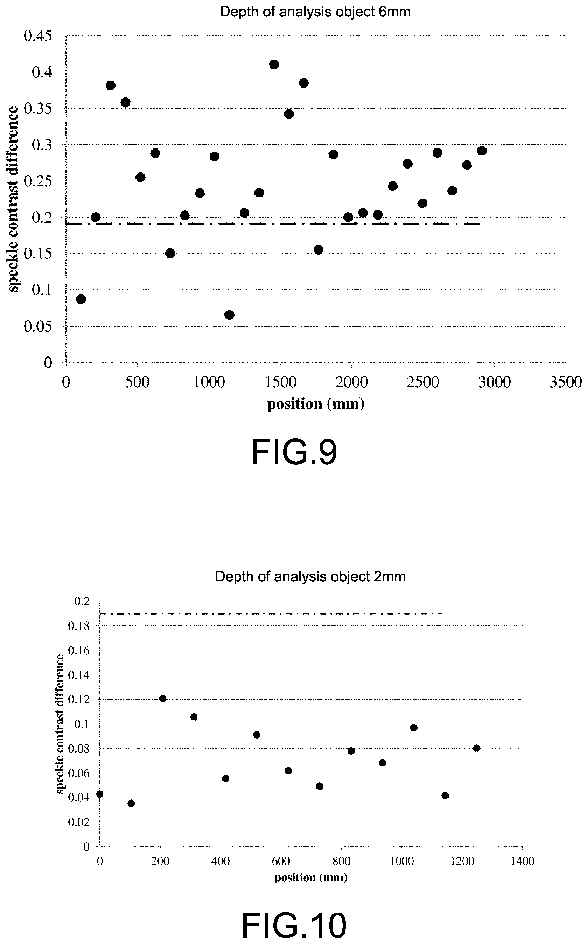

The imaging apparatus 2 according to the present technology includes the irradiation switching unit 21 that switches an irradiation method for the coherent light of the light irradiation unit 11.
Specifically, as shown in Fig. 8, in the imaging object O, in a manner that depends on a position of an object to be analyzed by using the speckles (hereinafter, also referred to as "analysis object"), i.e., a depth from the surface of the imaging object O to the analysis object, the irradiation method of the light irradiation unit 11 is switched from the spot irradiation to a method of irradiating the entire surface of the imaging object O with light (hereinafter, also referred to as "uniform irradiation").
More specifically, a description will be made with reference to Figs. 9 and 10. Fig. 9 shows speckle contrast due to the spot irradiation and the uniform irradiation in a case where the analysis object is located at a depth of 6 mm from the surface of the imaging object O. In Fig. 9, the vertical axis indicates speckle contrast and the horizontal axis indicates a distance from the irradiation position to the detection position. In addition, the long dashed short dashed line indicates the speckle contrast in the uniform irradiation and the plots indicate the speckle contrast in the spot irradiation.
As shown in Fig. 9, the numerical values of the speckle contrast due to the spot irradiation are higher than the numerical values of the speckle contrast due to the uniform irradiation.
Therefore, it can be seen that in a case where the distance from the surface of the imaging object O to the analysis object is 6 mm, the spot irradiation is more suitable for capturing the speckle image.
On the other hand, Fig. 10 shows speckle contrast due to the spot irradiation and the uniform irradiation in a case where the analysis object is located at a depth of 2 mm from the surface of the imaging object O. The vertical axis indicates the speckle contrast and the horizontal axis indicates the distance from the irradiation position to the detection position. In addition, the long dashed short dashed line indicates the speckle contrast in the uniform irradiation and the plots indicate the speckle contrast in the spot irradiation.

As shown in Fig. 10, the numerical values of the speckle contrast due to the uniform irradiation are higher than the numerical values of the speckle contrast due to the spot irradiation.

Therefore, it can be seen that in a case where the distance from the surface of the imaging object O to the analysis object is 2 mm, the uniform irradiation is more suitable for capturing the speckle image.

In view of the results of Figs. 9 and 10, the uniform irradiation is suitable in a case where the depth from the surface of the imaging object O to the analysis object is small and the spot irradiation is suitable in a case where the depth from the surface of the imaging object O to the analysis object is large.

Therefore, in a case where the analysis object is located at a deep position in the imaging object O, the irradiation switching unit 21 keeps the spot irradiation as the irradiation method of the light irradiation unit 11. In a case where the analysis object is located at a shallow position in the imaging object O, the irradiation switching unit 21 performs processing of switching to the uniform irradiation.

It should be noted that a switching method of the irradiation switching unit 21 is not particularly limited, and a well-known method can be employed. For example, there are a method projecting a light bulb such as liquid-crystal and the like.

In accordance with the thus configured imaging apparatus 2 according to the present technology, as in the imaging apparatus 1, the coherent light is propagated through a particular optical path in the imaging object O due to the spot irradiation of the light irradiation unit 11 and the light detection unit 12 detects the coherent light propagated through the optical path.

Therefore, even if the fluid contained in the imaging object O is located at a deep position in the imaging object O, it is possible to suitably image the fluid by using the speckles and to accurately analyze the state of the fluid.

In addition, the light irradiation unit 11 irradiates the plurality of positions of the imaging object O with light and the speckle image is generated on the basis of the optical information emitted from each of the detection positions. Therefore, it is possible to display the speckles in real time, and thus to obtain information (speed and the like) regarding the fluid at the deep portion with higher sensitivity and accuracy than typical uniform irradiation.

Moreover, the irradiation method can be switched by the irradiation switching unit 21 in a manner that depends on the position of the fluid in the imaging object O, and it is possible to obtain information (speed and the like) regarding the fluid at the deep portion with high sensitivity and accuracy irrespective of the position of the fluid. Thus, the adaptability is improved.

<3. Imaging Apparatus According to Third Embodiment>

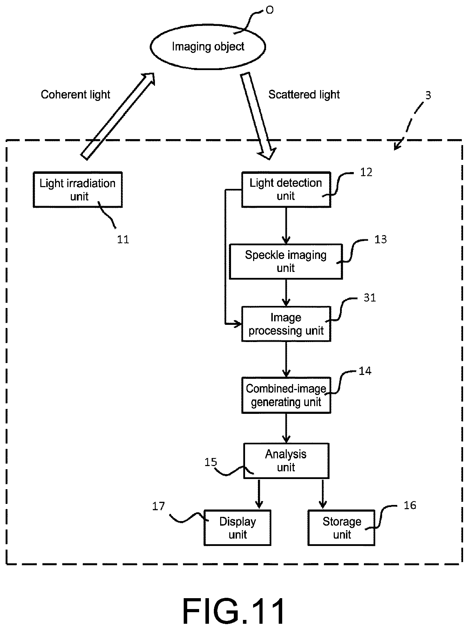

Next, a third embodiment of the imaging apparatus according to the present technology will be described with reference to Fig. 11.

An imaging apparatus 3 according to the third embodiment is different from the imaging apparatus 1 according to the first embodiment in that the imaging apparatus 3 according to the third embodiment includes an image processing unit 31. On the other hand, other configurations are identical to those of the imaging apparatus 1 according to the first embodiment, and thus will be denoted by identical signs and descriptions thereof will be omitted.

(1) Image Processing Unit

In the imaging apparatus according to the present technology, on the basis of electrical signals generated from the respective detection positions corresponding to the respective spot irradiation positions, a plurality of speckle images due to the respective spot irradiation are captured.

Therefore, due to some irradiation positions of the light irradiation unit 11, there is also a possibility that the fluid is not present on the optical path of the coherent light and the light detection unit 12 does not detect the optical information or the speckle image is not generated by the speckle imaging unit 13.

Thus, the imaging apparatus 3 according to the present technology includes the image processing unit 31 that determines whether or not light is detected by the light detection unit 12.

Specifically, the image processing unit 31 receives input of, for example, the detection result obtained by the light detection unit 12 or image information generated by the speckle imaging unit 13 on the basis of the respective detection positions, and selects and extracts, on the basis of it, information regarding the detection positions at which no speckles are generated.

Then, the image processing unit 31 outputs the extracted detection information to the combined-image generating unit 14 (see Fig. 11).

Note that a selection method and an extraction method for the detection information in this image processing unit 31 are not particularly limited, and well-known methods can be used.

Then, in the imaging apparatus 3 according to the present technology, the speckle combined image is generated at the combined-image generating unit 14 on the basis of the detection information selected and extracted by the image processing unit 31. Otherwise, as described above, the combined-image generating unit 14 is not a configuration necessarily provided in the imaging apparatus according to the present technology, and thus, in a case where the combined-image generating unit 14 is not provided, an image unsuitable for analysis is deleted, for example, on the basis of the information selected and extracted by the image processing unit 31.

In accordance with the thus configured imaging apparatus 3 according to the present technology, as in the imaging apparatus 1, the coherent light is propagated through a particular optical path in the imaging object O due to the spot irradiation of the light irradiation unit 11 and the light detection unit 12 detects the coherent light propagated through the optical path. Therefore, even if the fluid contained in the imaging object O is located at a deep position in the imaging object O, it is possible to suitably image the fluid by using the speckles and to accurately analyze the state of the fluid.

In addition, the light irradiation unit 11 irradiates the plurality of positions of the imaging object O with light and the speckle image is generated on the basis of the optical information emitted from each of the detection positions. Therefore, it is possible to display the speckles in real time, and thus to obtain information (speed and the like) regarding the fluid at the deep portion with higher sensitivity and accuracy than typical uniform irradiation.

Furthermore, due to the provision of the image processing unit 31, the quality of the speckle image can be improved, and information (speed and the like) regarding the fluid at the deep portion can still be obtained with high sensitivity and accuracy.

It should be noted that although the imaging apparatus 3 according to the present technology shown in Fig. 11 does not include the irradiation switching unit 21 provided in the imaging apparatus 2 according to the second embodiment, the imaging apparatus 3 may include, as necessary, the irradiation switching unit 21.

<4. Imaging Apparatus According to Fourth Embodiment>

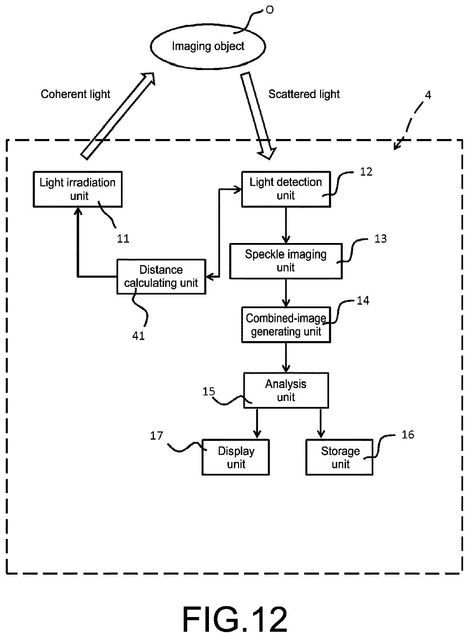

Next, a fourth embodiment of the imaging apparatus according to the present technology will be described with reference to Fig. 12.

An imaging apparatus 4 according to the fourth embodiment is different from the imaging apparatus 1 according to the first embodiment in that the imaging apparatus 4 according to the fourth embodiment includes a distance calculating unit 41. On the other hand, other configurations are identical to those of the imaging apparatus 1 according to the first embodiment, and thus will be denoted by identical signs and descriptions thereof will be omitted.

(1) Distance Calculating Unit

As described above, the coherent light emitted from the light irradiation unit 11 is propagated through the optical path represented by Expression 3 in the imaging object O. Then, the coherent light propagated through the optical path is detected by the light detection unit 12. Thus, the optical information of the imaging object O and/or the fluid contained in the same is converted into electrical signals.

In other words, it is possible to determine whether or not the fluid that is the analysis object is present on the optical path by using the optical signals detected by the light detection unit 12, and thus, it is possible to calculate a distance r from the irradiation position to the detection position on the basis of the information according to Expression 3.

Then, the calculated distance r is output to the light irradiation unit 11 and/or the light detection unit 12.

That is, the distance calculating unit 41 receives input of the detection information acquired by the light detection unit 12. The distance calculating unit 41 is capable of calculating, on the basis of this detection information, a distance r suitable for analyzing the state of the imaging object O and/or the fluid contained in the same. As a result, a spot irradiation pattern suitable for analyzing the imaging object O can also be generated.

In other words, a suitable detection position with respect to the irradiation position of the coherent light and a suitable irradiation position of the coherent light with respect to the detection position can be calculated.

It should be noted that in accordance with the imaging apparatus 4 according to the present technology, a distance from the surface of the imaging object O to the fluid can also be calculated by using Expressions 3 and 4.

In accordance with the thus configured imaging apparatus 4 according to the present technology, as in the imaging apparatus 1, the coherent light is propagated through a particular optical path in the imaging object O due to the spot irradiation of the light irradiation unit 11 and the light detection unit 12 detects the coherent light propagated through the optical path. Therefore, even if the fluid contained in the imaging object O is located at a deep position in the imaging object O, it is possible to suitably image the fluid by using the speckles and to accurately analyze the state of the fluid.

In addition, the light irradiation unit 11 irradiates the plurality of positions of the imaging object O with light and the speckle image is generated on the basis of the optical information emitted from each of the detection positions. Therefore, it is possible to display the speckles in real time, and thus to obtain information (speed and the like) regarding the fluid at the deep portion with higher sensitivity and accuracy than typical uniform irradiation.

Moreover, due to the provision of the distance calculating unit 41, it is possible to calculate an irradiation position of the light irradiation unit 11 or a detection position of the light detection unit 12, which is suitable for analyzing the imaging object O and/or the fluid contained in the same. Therefore, information (speed and the like) regarding the fluid at the deep portion can be obtained with higher sensitivity and accuracy.

It should be noted that although the imaging apparatus 4 according to the present technology shown in Fig. 12 does not include the irradiation switching unit 21, which is provided in the imaging apparatus 2 according to the second embodiment, and the image processing unit 31 according to the third embodiment, the imaging apparatus 4 may include, as necessary, the irradiation switching unit 21 and the image processing unit 31.

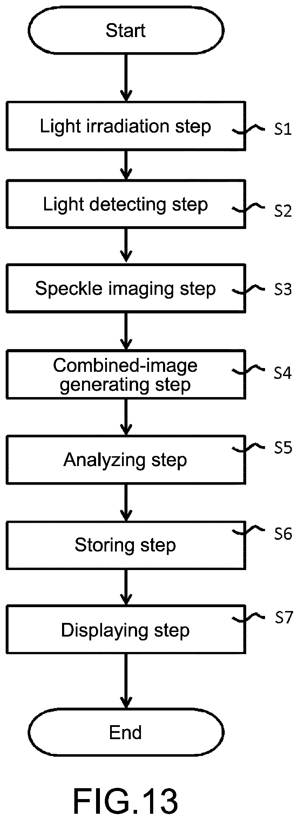

The present technology also provides an imaging method. Hereinafter, the imaging method according to the present technology will be described with reference to Figs. 13 to 16.

<5. Imaging Method According to First Embodiment>

An imaging method according to the first embodiment at least includes a light irradiation step S1, a light detecting step S2, and a speckle imaging step S3, and may include, as necessary, a combined-image generating step S4, an analyzing step S5, a storing step S6, and a displaying step S7. Hereinafter, each of the steps will be described.

(1) Light Irradiation Step

An imaging method according to the first embodiment includes a step of irradiating the imaging object O with the coherent light from the light source.

As the light source used in this light irradiation step S1, for example, an argon ion (Ar) laser, a helium-neon (He-Ne) laser, a dye laser, a krypton (Cr) laser, a distributed feedback (DFB) or grating feedback semiconductor laser, and the like can be used.

Although the wavelength number of the coherent light emitted in the light irradiation step S1 is not particularly limited, and can be changed as appropriate in a manner that depends on the types of the imaging object O and/or the fluid contained in the same. For example, in a case where the fluid is blood flowing in a blood vessel, it is favorable that the wavelength number of the coherent light is within a range of 600 to 1550 nm and it is red light that provides a low light absorption rate.

In addition, the light irradiation step S1 according to the present technology includes irradiating the plurality of positions of the imaging object O with coherent light as spots.

Note that in a case where the coherent light is detected at a point different from the irradiation position, a most likely optical path through which it can arrive at the detection position is represented by Expression 7 as follows.

[Expression 7]

$$Z(x) \approx \sqrt{\frac{1}{8}\left\{\sqrt{\left[x^2+(r-x)^2\right]^2+32x^2(r-x)^2}-x^2-(r-x)^2\right\}}$$

$$r\sqrt{3\mu_a\mu_s(1-g)} << 1$$

In Expression 7, "r" denotes a distance from the irradiation position to the detection position, "$\mu_a$" denotes an equivalent absorption coefficient, "$\mu_s$" denotes an equivalent scattering coefficient, and G denotes an anisotropy coefficient.

Further, a depth when the coherent light enters most deeply in the optical path represented by Expression 7 is represented by Expression 8 as follows.

[Expression 8]

$$Z_{depth} = \frac{\sqrt{2}}{4} r$$

Note that in a case where the coherent light is propagated in a portion of the imaging object O, which is deeper than the fluid, there is a possibility that optical signals from portions of the imaging object O other than the fluid are detected as noise.

Thus, it is favorable that the optical path of the coherent light with which the imaging object O is irradiated in the light irradiation step S1 is an optical path represented by Expression 7, whose deepest portion through which the coherent light is propagated is associated with the fluid.

Moreover, in the light irradiation step S1, the pattern formed by the plurality of spot irradiation positions may be, for example, a state in which a plurality of spot irradiation positions are regularly arrayed or may be arranged in spots on the imaging object O.

As a method of generating this pattern, the method of projecting the pattern by using the image formation of the lens (see Fig. 5), the method of projecting the pattern by using the holography (see Fig. 6), the method of projecting the pattern by using the Talbot effect (see Fig. 7), which are described above, and the like can be exemplified.

(2) Light Detecting Step

In the imaging method according to the present technology, the light detecting step S2 is performed after the light irradiation step S1 is performed.

Specifically, optical signals based on scattered light emitted from the imaging object O due to coherent light propagated in the imaging object O are detected and converted into electrical signals.

It should be noted that a method of converting the optical signals into the electrical signals is not particularly limited, and a well-known method can be used.

(3) Speckle Imaging Step

The imaging method according to the present technology includes the speckle imaging step S3 of capturing a speckle image on the basis of the scattered light obtained in the light irradiation step S1.

Specifically, a plurality of speckle images due to the respective spot irradiation positions are captured on the basis of electrical signals generated from the detection positions corresponding to the respective spot irradiation positions.

An imaging method in this speckle imaging step S3 is not particularly limited, and one type or two types or more of well-known imaging methods may be selected and used freely in combination. For example, there are imaging methods using imaging elements such as a charge coupled device (CCD), a CMOS sensor of a global shutter system, and a CMOS sensor of a rolling shutter system.

(4) Combined-Image Generating Step

The imaging method according to the present technology may include, as necessary, the combined-image generating step S4.

In this combined-image generating step S4, for example, a plurality of speckle images are combined in accordance with a well-known method such as a method of generating one speckle combined image by making image information (e.g., the number of pixels, the color tone of pixels, and the like) of the respective speckle images the same and a method of simply making the respective speckle images overlap each other.

It should be noted that the combined image generated in the combined-image generating step S4 needs to be generated such that the state of the imaging object O can be analyzed on the basis of the generated combined image. Therefore, in the combined-image generating step S4, for example, a method of correcting, on the basis of luminance values of each speckle image, the luminance values of the speckle combined image such that the luminance distribution in the speckle combined image becomes uniform and the like may be performed.

(5) Analyzing Step

The imaging method according to the present technology may include, as necessary, the analyzing step S5.

In this analyzing step S5, for example, an intensity distribution of speckles is measured by using a luminance meter and the like on a speckle image captured in the speckle imaging step S3 or the combined image generated in the combined-image generating step S4. Using a result of the measurement, speckle contrast, which is a value obtained by dividing the standard deviation of the intensity distribution by the average of the intensity distribution, is measured.

By such measurement of speckle contrast, angiography can be performed by using a change in speckle contrast in a case where the imaging object O is assumed as a blood vessel being a light scattering fluid. Furthermore, since the speckles vary with time, the speed of the blood flow can also be analyzed.

It should be noted that a method of measuring the intensity distribution of the speckles or the speckle contrast is not particularly limited as long as the effect of the present technology is not impaired, and one type or two types or more of well-known measurement methods may be selected and used freely in combination.

(6) Storing Step

The imaging method according to the present technology may include, as necessary, the storing step S6.

In this storing step S6, the speckle image captured in the speckle imaging step S3, the combined image generated in the combined-image generating step S4, the speckle contrast measured in the analyzing step S5, the analysis result in the analyzing step S5, and the like are stored.

(7) Displaying Step

The imaging method according to the present technology may include, as necessary, the displaying step S7. In this displaying step S7, the speckle image captured in the speckle imaging step S3, the combined image generated in the combined-image generating step S4, the analysis result in the analyzing step S5, and the like are displayed on, for example, a monitor or the like.

In accordance with the imaging method including the above-mentioned steps according to the present technology, the coherent light is propagated through a particular optical path in the imaging object O due to the spot irradiation in the light irradiation step S1 and the coherent light propagated through the optical path is detected.

Therefore, even if the fluid contained in the imaging object O is located at a deep position in the imaging object O, it is possible to suitably image the fluid by using the speckles and to accurately analyze the state of the fluid.

In addition, the plurality of positions of the imaging object O are irradiated with light in the light irradiation step S1 and the speckle image is generated on the basis of the optical information emitted from each of the detection positions. Therefore, it is possible to display the speckles in real time, and thus to obtain information (speed and the like) regarding the fluid at the deep portion with higher sensitivity and accuracy than typical uniform irradiation.

<6. Imaging Method According to Second Embodiment>

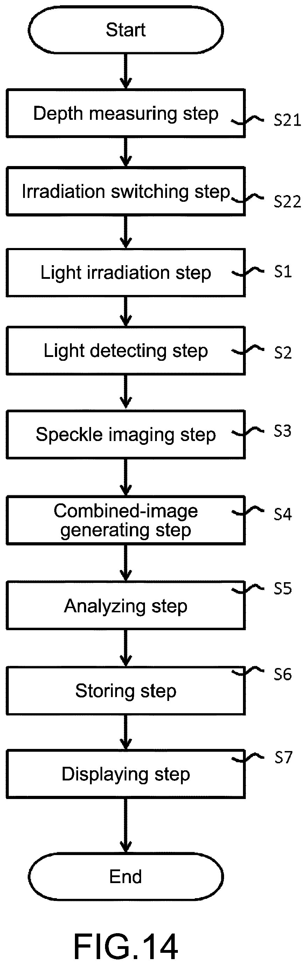

Next, the second embodiment of the imaging method according to the present technology will be described with reference to Fig. 14.

As in the imaging method according to the first embodiment, the imaging method according to the second embodiment at least includes the light irradiation step S1, the light detecting step S2, and the speckle imaging step S3, and may include, as necessary, the combined-image generating step S4, the analyzing step S5, the storing step S6, and the displaying step S7.

On the other hand, the imaging method according to the second embodiment is different from the imaging method according to the first embodiment in that the imaging method according to the second embodiment includes a depth measuring step S21 and an irradiation switching step S22.

Hereinafter, the depth measuring step S21 and the irradiation switching step S22, which are different from the imaging method according to the first embodiment, will be described, and common steps will be denoted by identical signs and descriptions thereof will be omitted.

(1) Depth Measuring Step

The imaging method according to the second embodiment includes the depth measuring step S21. In this depth measuring step S21, a distance (depth) from the surface of the imaging object O to an object to be analyzed by using the speckles (hereinafter, also referred to as "analysis object") is measured.

A measuring method of in this depth measuring step S21 is not particularly limited, and a well-known method can be employed. For example, there are a method of measuring the depth by seeing the imaging object O, a method of measuring the depth on the basis of a result obtained by detecting the coherent light propagated through the optical path according to Expression 7 in advance, and the like.

(2) Irradiation Switching Step

In the imaging method of the present technology shown in Fig. 14, after the depth of the analysis object is measured in the depth measuring step S21, switching of the irradiation method for the coherent light is performed on the basis of a result of the measurement (irradiation switching step S22).

Note that as described above, the uniform irradiation is more suitable in a case where the depth from the surface of the imaging object O to the analysis object is small, and the spot irradiation is more suitable in a case where the depth from the surface of the imaging object O to the analysis object is large.

Therefore, in the irradiation switching step S22, in a case where the depth of the analysis object is measured as a depth equal to a predetermined depth or larger than the predetermined depth in the depth measuring step S21, switching is performed such that the spot irradiation is performed on the imaging object O in the light irradiation step S1.

On the other hand, in a case where the depth of the analysis object is measured as a depth smaller than the predetermined depth in the depth measuring step S21, switching is performed such that the uniform irradiation is performed on the imaging object O in the light irradiation step S1.

It should be noted that a switching method in the irradiation switching step S22 is not particularly limited, and a well-known method can be employed. For example, there are a method projecting a light bulb such as liquid-crystal and the like.

In accordance with the imaging method including the above-mentioned steps according to the second embodiment, the coherent light is propagated through a particular optical path in the imaging object O due to the spot irradiation in the light irradiation step S1 and the coherent light propagated through the optical path is detected.

Therefore, even if the fluid contained in the imaging object O is located at a deep position in the imaging object O, it is possible to suitably image the fluid by using the speckles and to accurately analyze the state of the fluid.

In addition, the plurality of positions of the imaging object O are irradiated with light in the light irradiation step S1 and the speckle image is generated on the basis of the optical information emitted from each of the detection positions. Therefore, it is possible to display the speckles in real time, and thus to obtain information (speed and the like) regarding the fluid at the deep portion with higher sensitivity and accuracy than typical uniform irradiation.

Furthermore, in the irradiation switching step S22, the irradiation method can be switched in a manner that depends on the position of the fluid in the imaging object O, and it is possible to obtain information (speed and the like) regarding the fluid at the deep portion with high sensitivity and accuracy irrespective of the position of the fluid. Thus, the adaptability is improved.

<7. Imaging Method According to Third Embodiment>

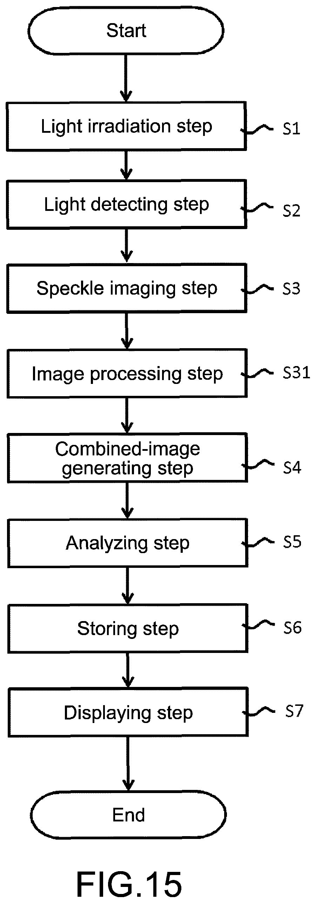

Next, a third embodiment of the imaging method according to the present technology will be described with reference to Fig. 15. As in the imaging method according to the first embodiment, the imaging method according to the third embodiment at least includes the light irradiation step S1, the light detecting step S2, and the speckle imaging step S3, and may include, as necessary, the combined-image generating step S4, the analyzing step S5, the storing step S6, and the displaying step S7.

On the other hand, the imaging method according to the third embodiment is different from the imaging method according to the first embodiment in that the imaging method according to the third embodiment includes an image processing step S31.

Hereinafter, the image processing step S31, which is different from the imaging method according to the first embodiment, will be described, and common steps will be denoted by identical signs and descriptions thereof will be omitted.

(1) Image Processing Step

In the imaging method according to the present technology shown in Fig. 15, in the speckle imaging step S3, a plurality of speckle images due to the respective spot irradiation are captured on the basis of electrical signals generated from the detection positions corresponding to the respective spot irradiation positions.

Therefore, due to some irradiation positions in the light irradiation step S1, there is also a possibility that the fluid is not present on the optical path of the coherent light and the optical information is not detected in the light detecting step S2 or the speckle image is not generated in the speckle imaging step S3.

Thus, the imaging method according to the third embodiment includes the image processing step S31 in which whether or not light is detected in the light detecting step S2 is determined.

In this image processing step S31, on the basis of the image information generated on the basis of the respective detection positions or detected optical information in the speckle imaging step S3, information based on the detection positions at which no speckles are generated is selected and extracted.

It should be noted that a selection method and an extraction method for the information in this image processing step S31 is not particularly limited, and well-known methods can be used.

Then, in the imaging method according to the third embodiment, on the basis of the detection information selected and extracted in the image processing step S31, the speckle combined image is generated in the combined-image generating step S4.

Alternatively, in a case where the combined-image generating step S4 that is not an essential configuration is not provided, an image unsuitable for analysis is deleted, for example, on the basis of the detection information selected and extracted in the image processing step S31.

In accordance with the imaging method including the above-mentioned steps according to the third embodiment, the coherent light is propagated through a particular optical path in the imaging object O due to the spot irradiation in the light irradiation step S1 and the coherent light propagated through the optical path is detected.

Therefore, even if the fluid contained in the imaging object O is located at a deep position in the imaging object O, it is possible to suitably image the fluid by using the speckles and to accurately analyze the state of the fluid.

In addition, the plurality of positions of the imaging object O are irradiated with light in the light irradiation step S1 and the speckle image is generated on the basis of the optical information emitted from each of the detection positions. Therefore, it is possible to display the speckles in real time, and thus to obtain information (speed and the like) regarding the fluid at the deep portion with higher sensitivity and accuracy than typical uniform irradiation.

Furthermore, due to the provision of the image processing step S31, the quality of the speckle image can be improved, and information (speed and the like) regarding the fluid at the deep portion can still be obtained with high sensitivity and accuracy.

It should be noted that although the imaging method according to the present technology shown in Fig. 15 does not include the depth measuring step S21 and the irradiation switching step S22, which are provided in the imaging method according to the second embodiment, the imaging method according to the third embodiment may include, as necessary, the depth measuring step S21 and the irradiation switching step S22.

<8. Imaging Method According to Fourth Embodiment>

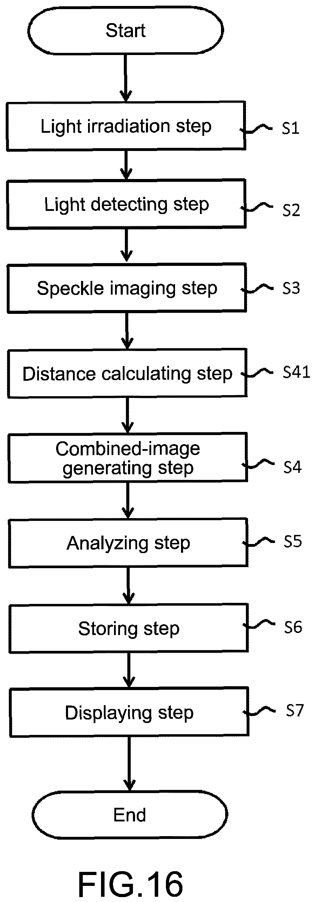

Next, the fourth embodiment of the imaging method according to the present technology will be described with reference to Fig. 16.

As in the imaging method according to the first embodiment, the imaging method according to the fourth embodiment at least includes the light irradiation step S1, the light detecting step S2, and the speckle imaging step S3, and may include, as necessary, the combined-image generating step S4, the analyzing step S5, the storing step S6, and the displaying step S7.

On the other hand, the imaging method according to the fourth embodiment is different from the imaging method according to the first embodiment in that the imaging method according to the fourth embodiment includes the distance calculating step S41.

Hereinafter, the distance calculating step S41, which is different from the imaging method according to the first embodiment, will be described, and common steps will be denoted by identical signs and descriptions thereof will be omitted.

(1) Distance Calculating Step

In the imaging method according to the present technology, the emitted coherent light is propagated through the optical path represented by Expression 7. Then, on the basis of the coherent light propagated through the optical path, optical characteristics of the imaging object O and/or the fluid contained in the same are converted into electrical signals.

In other words, it is possible to determine whether or not the fluid that is the analysis object is present on the optical path on the basis of the information detected by the light detection unit 12, and thus, it is possible to calculate a distance r from the irradiation position to the detection position on the basis of the information according to Expression 7.

That is, due to the provision of the distance calculating step S41, it is possible to calculate, on the basis of the detection result obtained in the light detecting step S2, a distance r suitable for analyzing the state of the imaging object O and/or the fluid contained in the same.

In other words, a suitable detection position with respect to the irradiation position of the coherent light and a suitable irradiation position of the coherent light with respect to the detection position can be calculated.

It should be noted that in accordance with the imaging method according to the present technology, a distance from the surface of the imaging object O to the fluid can also be calculated by using the characteristics of the detected signals and Expressions 7 and 8.

Moreover, in a case where the imaging method according to the fourth embodiment includes the storing step S6 and the displaying step S7, the distance from the irradiation position to the detection position, which is calculated in the distance calculating step S41, may be stored and displayed.

In accordance with such an imaging method according to the present technology, the coherent light is propagated through a particular optical path in the imaging object O due to the spot irradiation in the light irradiation step S1 and the coherent light propagated through the optical path is detected.

Therefore, even if the fluid contained in the imaging object O is located at a deep position in the imaging object O, it is possible to suitably image the fluid by using the speckles and to accurately analyze the state of the fluid.

In addition, the plurality of positions of the imaging object O are irradiated with light in the light irradiation step S1 and the speckle image is generated on the basis of the optical information emitted from each of the detection positions. Therefore, it is possible to display the speckles in real time, and thus to obtain information (speed and the like) regarding the fluid at the deep portion with higher sensitivity and accuracy than typical uniform irradiation.

Moreover, due to the provision of the distance calculating step S41, it is possible to calculate an irradiation position obtained in the light irradiation step S1 or a detection position obtained in the light detecting step S2, which is suitable for analyzing the imaging object O and/or the fluid contained in the same. Therefore, information (speed and the like) regarding the fluid at the deep portion can be obtained with higher sensitivity and accuracy.

It should be noted that although the imaging method according to the present technology shown in Fig. 16 does not include the depth measuring step S21 and the irradiation switching step S22, which are provided in the imaging method according to the second embodiment, and the image processing step S31 provided in the imaging method according to the third embodiment, and the imaging method according to the fourth embodiment may include, as necessary, the depth measuring step S21, the irradiation switching step S22, and the image processing step S31.

It should be noted that the imaging apparatus according to the present technology can also take the following configurations.

(1) An imaging apparatus, including:

a light irradiation unit that irradiates a plurality of positions of an imaging object with coherent light as spots;
a light detection unit that detects light, the light being emitted from the light irradiation unit and propagated in the imaging object; and
an imaging unit that captures a speckle image obtained from scattered light of the imaging object, the scattered light being detected by the light detection unit.

(2) The imaging apparatus according to (1), in which
the light irradiation unit irradiates the plurality of positions of the imaging object with light as spots in such a manner that the spots are arranged at predetermined intervals on the imaging object.

(3) The imaging apparatus according to (1) or (2), in which
an optical path of the light propagated in the imaging object is represented by Expression 9 as follows.

[Expression 9]

$$Z(x) \approx \sqrt{\frac{1}{8}\left\{\sqrt{\left[x^2 + (r-x)^2\right]^2 + 32x^2(r-x)^2} - x^2 - (r-x)^2\right\}}$$

$$r\sqrt{3\mu_a\mu_s(1-g)} << 1$$

(4) The imaging apparatus according to any one of (1) to (3), further including
an image processing unit that determines whether or not light is detected by the light detection unit on a basis of imaging information obtained by the imaging unit.

(5) The imaging apparatus according to (3) or (4), further including
a distance calculating unit that calculates, on a basis of Expression 9, a distance between a spot irradiation position of the light irradiation unit and a detection position of the light propagated in the imaging object.

(6) The imaging apparatus according to any one of (1) to (5), further including
an irradiation switching unit that switches an irradiation method of the light irradiation unit from spot irradiation to uniform irradiation.

(7) The imaging apparatus according to any one of (1) to (6), further including

a combined-image generating unit that generates a combined image on a basis of a plurality of pieces of light information detected by the light detection unit.

Moreover, the imaging method according to the present technology can also take the following configurations.

(8) An imaging method, including:

a light irradiation step of irradiating a plurality of positions of an imaging object with coherent light as spots;
a light detecting step of detecting light, the light being emitted as spots and propagated in the imaging object; and
an imaging step of capturing a speckle image obtained from scattered light of the imaging object, the scattered light being detected in the light detecting step.

(9) The imaging method according to (8), in which
the light irradiation step includes emitting the coherent light to the plurality of positions of the imaging object in such a manner that light beams of the coherent light are arranged at predetermined intervals on the imaging object.
(10) The imaging method according to (8) or (9), in which
the light propagated in the imaging object is represented by Expression 10 as follows.

[Expression 10]

$$Z(x) \approx \sqrt{\frac{1}{8}\left\{\sqrt{\left[x^2 + (r-x)^2\right]^2 + 32x^2(r-x)^2} - x^2 - (r-x)^2\right\}}$$

$$r\sqrt{3\mu_a\mu_s(1-g)} \ll 1$$

(11) The imaging method according to any one of (8) to (10), further including
an image processing step of determining whether or not light is detected in the light detecting step on a basis of imaging information obtained in the imaging step.
(12) The imaging method according to (10) or (11), further including
a distance calculating step of calculating, on a basis of Expression 10, a distance between a spot irradiation position in the light irradiation step and a light detection position in the light detecting step.
(13) The imaging method according to any one of (8) to (12), further including
an irradiation switching step of switching an irradiation method for the coherent light from spot irradiation to uniform irradiation.
(14) The imaging method according to any one of (8) to (13), further including
a combined-image generating step of generating a combined image on a basis of a plurality of pieces of light information detected in the light detecting step. Reference Signs List

| 1, 2, 3, 4 | imaging apparatus |
| 11 | light irradiation unit |
| 12 | light detection unit |
| 13 | speckle imaging unit |
| 14 | combined-image generating unit |
| 15 | analysis unit |
| 16 | storage unit |
| 17 | display unit |
| O | imaging object |

**Claims**

1. An imaging apparatus, comprising:

a light irradiation unit that irradiates a plurality of positions of an imaging object with coherent light as spots;
a light detection unit that detects light, the light being emitted from the light irradiation unit and propagated in the imaging object; and
an imaging unit that captures a speckle image obtained from scattered light of the imaging object, the scattered light being detected by the light detection unit.

2. The imaging apparatus according to claim 1, wherein
the light irradiation unit irradiates the plurality of positions of the imaging object with light as spots in such a manner that the spots are arranged at predetermined intervals on the imaging object.

3. The imaging apparatus according to claim 2, wherein
an optical path of the light propagated in the imaging object is represented by Expression 1 as follows:

[Expression 1]

$$Z(x) \approx \sqrt{\frac{1}{8}\left\{\sqrt{\left[x^2 + (r-x)^2\right]^2 + 32x^2(r-x)^2} - x^2 - (r-x)^2\right\}}$$

$$r\sqrt{3\mu_a\mu_s(1-g)} << 1$$

4. The imaging apparatus according to claim 3, further comprising
an image processing unit that determines whether or not light is detected by the light detection unit on a basis of imaging information obtained by the imaging unit.

5. The imaging apparatus according to claim 4, further comprising
a distance calculating unit that calculates, on a basis of Expression 1, a distance between a spot irradiation position of the light irradiation unit and a detection position of the light propagated in the imaging object.

6. The imaging apparatus according to claim 5, further comprising
an irradiation switching unit that switches an irradiation method of the light irradiation unit from spot irradiation to uniform irradiation.

7. The imaging apparatus according to claim 6, further comprising
a combined-image generating unit that generates a combined image on a basis of a plurality of pieces of light information detected by the light detection unit.

8. An imaging method, comprising:

a light irradiation step of irradiating a plurality of positions of an imaging object with coherent light as spots;
a light detecting step of detecting light, the light being emitted as spots and propagated in the imaging object; and
an imaging step of capturing a speckle image obtained from scattered light of the imaging object, the scattered light being detected in the light detecting step.

9. The imaging method according to claim 8, wherein
the light irradiation step includes emitting the coherent light to the plurality of positions of the imaging object in such a manner that light beams of the coherent light are arranged at predetermined intervals on the imaging object.

10. The imaging method according to claim 9, wherein
the light propagated in the imaging object is represented by Expression 2 as follows:

[Expression 2]

$$Z(x) \approx \sqrt{\frac{1}{8}\left\{\sqrt{\left[x^2 + (r-x)^2\right]^2 + 32x^2(r-x)^2} - x^2 - (r-x)^2\right\}}$$

$$r\sqrt{3\mu_a\mu_s(1-g)} << 1$$

.

11. The imaging method according to claim 10, further comprising
an image processing step of determining whether or not light is detected in the light detecting step on a basis of imaging information obtained in the imaging step.

12. The imaging method according to claim 11, further comprising
a distance calculating step of calculating, on a basis of Expression 2, a distance between a spot irradiation position in the light irradiation step and a light detection position in the light detecting step.

13. The imaging method according to claim 12, further comprising
an irradiation switching step of switching an irradiation method for the coherent light from spot irradiation to uniform irradiation.

14. The imaging method according to claim 13, further comprising
a combined-image generating step of generating a combined image on a basis of a plurality of pieces of light information detected in the light detecting step.

FIG.1

FIG.2

FIG.3

FIG.4

(a) Coherent light
Light bulb
Projection lens
Imaging object
O

Display pattern

Projection pattern

(b) Spot irradiation

(c) Uniform irradiation

# FIG.5

Coherent light
Wavefront
Spatial light modulator
Imaging object
O

(a)

Display pattern (phase or intensity)

Projection pattern (intensity)

(b) Spot irradiation

(c) Uniform irradiation

# FIG.6

(a)

Coherent light

Wavefront  Spatial light modulator

Imaging object

O

Display pattern
(intensity)

Projection pattern
(intensity)

(b)  Spot
irradiation

(c)  Uniform
irradiation

FIG.7

FIG.8

Depth of analysis object 6mm

FIG.9

Depth of analysis object 2mm

FIG.10

O

Imaging object

Coherent light      Scattered light                    3

Light irradiation
unit                    Light detection              12
                        unit

            11          Speckle imaging              13
                        unit

                        Image                        31
                        processing unit

                        Combined-image               14
                        generating unit

                        Analysis
            15          unit

17    Display                    Storage              16
      unit                       unit

# FIG.11

O

Imaging object

Coherent light          Scattered light          4

Light irradiation unit

Light detection unit          12

11

Speckle imaging unit          13

Distance calculating unit

Combined-image generating unit          14

41

Analysis unit

15

17          Display unit          Storage unit          16

# FIG.12

```
            ┌──────────────┐
            │    Start     │
            └──────┬───────┘
                   │
                   ▼
        ┌────────────────────┐
        │ Light irradiation step │ ～ S1
        └──────┬─────────────┘
                   │
                   ▼
        ┌────────────────────┐
        │  Light detecting step  │ ～ S2
        └──────┬─────────────┘
                   │
                   ▼
        ┌────────────────────┐
        │ Speckle imaging step │ ～ S3
        └──────┬─────────────┘
                   │
                   ▼
        ┌────────────────────┐
        │   Combined-image     │ ～ S4
        │   generating step    │
        └──────┬─────────────┘
                   │
                   ▼
        ┌────────────────────┐
        │    Analyzing step     │ ～ S5
        └──────┬─────────────┘
                   │
                   ▼
        ┌────────────────────┐
        │     Storing step      │ ～ S6
        └──────┬─────────────┘
                   │
                   ▼
        ┌────────────────────┐
        │    Displaying step    │ ～ S7
        └──────┬─────────────┘
                   │
                   ▼
            ┌──────────────┐
            │     End      │
            └──────────────┘
```

# FIG.13

```
        ┌──────────────┐
        │    Start     │
        └──────┬───────┘
               ↓
        ┌──────────────────────┐
        │ Depth measuring step  │∼ S21
        └──────┬───────────────┘
               ↓
        ┌──────────────────────────┐
        │ Irradiation switching step│∼ S22
        └──────┬───────────────────┘
               ↓
        ┌──────────────────────┐
        │ Light irradiation step│∼ S1
        └──────┬───────────────┘
               ↓
        ┌──────────────────────┐
        │  Light detecting step │∼ S2
        └──────┬───────────────┘
               ↓
        ┌──────────────────────┐
        │ Speckle imaging step  │∼ S3
        └──────┬───────────────┘
               ↓
        ┌──────────────────────┐
        │    Combined-image     │∼ S4
        │   generating step     │
        └──────┬───────────────┘
               ↓
        ┌──────────────────────┐
        │    Analyzing step     │∼ S5
        └──────┬───────────────┘
               ↓
        ┌──────────────────────┐
        │     Storing step      │∼ S6
        └──────┬───────────────┘
               ↓
        ┌──────────────────────┐
        │   Displaying step     │∼ S7
        └──────┬───────────────┘
               ↓
        ┌──────────────┐
        │     End      │
        └──────────────┘
```

# FIG.14

```
              ┌──────────────┐
              │    Start     │
              └──────────────┘
                     │
                     ▼
         ┌────────────────────────┐
         │  Light irradiation step │ ～ S1
         └────────────────────────┘
                     │
                     ▼
         ┌────────────────────────┐
         │   Light detecting step  │ ～ S2
         └────────────────────────┘
                     │
                     ▼
         ┌────────────────────────┐
         │   Speckle imaging step  │ ～ S3
         └────────────────────────┘
                     │
                     ▼
         ┌────────────────────────┐
         │  Image processing step  │ ～ S31
         └────────────────────────┘
                     │
                     ▼
         ┌────────────────────────┐
         │     Combined-image      │ ～ S4
         │     generating step     │
         └────────────────────────┘
                     │
                     ▼
         ┌────────────────────────┐
         │     Analyzing step      │ ～ S5
         └────────────────────────┘
                     │
                     ▼
         ┌────────────────────────┐
         │      Storing step       │ ～ S6
         └────────────────────────┘
                     │
                     ▼
         ┌────────────────────────┐
         │     Displaying step     │ ～ S7
         └────────────────────────┘
                     │
                     ▼
              ┌──────────────┐
              │     End      │
              └──────────────┘
```

FIG.15

Start

Light irradiation step — S1

Light detecting step — S2

Speckle imaging step — S3

Distance calculating step — S41

Combined-image generating step — S4

Analyzing step — S5

Storing step — S6

Displaying step — S7

End

FIG.16

## INTERNATIONAL SEARCH REPORT

| | International application No. |
|---|---|
| | PCT/JP2017/001858 |

### A. CLASSIFICATION OF SUBJECT MATTER
*G01N21/17(2006.01)i*

According to International Patent Classification (IPC) or to both national classification and IPC

### B. FIELDS SEARCHED

Minimum documentation searched (classification system followed by classification symbols)
G01N21/00, 21/01, 21/17-21/61, A61B3/00-3/18, 5/06-5/22, 10/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
JSTPlus/JMEDPlus/JST7580(JDreamIII)

### C. DOCUMENTS CONSIDERED TO BE RELEVANT

| Category* | Citation of document, with indication, where appropriate, of the relevant passages | Relevant to claim No. |
|---|---|---|
| X<br>A | JP 2010-503475 A  (The General Hospital Corp.),<br>04 February 2010 (04.02.2010),<br>paragraphs [0002], [0015] to [0017], [0025] to [0028], [0035]<br>& US 2008/0287808 A1<br>paragraphs [0002], [0029] to [0031], [0039] to [0042], [0049]<br>& WO 2008/033909 A2    & EP 2077753 A1 | 1-5,8-12<br>6,7,13,14 |

| ☒  Further documents are listed in the continuation of Box C. | ☐  See patent family annex. |
|---|---|

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered    to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 27 March 2017 (27.03.17) | 11 April 2017 (11.04.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | |
| | Telephone No. |

Form PCT/ISA/210 (second sheet) (January 2015)

**INTERNATIONAL SEARCH REPORT**

International application No.

PCT/JP2017/001858

C (Continuation).  DOCUMENTS CONSIDERED TO BE RELEVANT

| Category* | Citation of document, with indication, where appropriate, of the relevant passages | Relevant to claim No. |
|---|---|---|
| Y<br>A | WO 2014/116483 A1  (NANYANG TECHNOLOGICAL UNIVERSITY),<br>31 July 2014 (31.07.2014),<br>paragraphs [0031] to [0034], [0037], [0038], [0040]; fig. 3B, 5<br>& JP 2016-509509 A      & US 2014/0206980 A1<br>& EP 2948052 A1        & CA 2898708 A<br>& AU 2014209741 A       & CN 105188523 A<br>& KR 10-2015-0135241 A   & MX 2015009381 A | 1-5,8-12<br>6,7,13,14 |
| Y | JP 2005-515818 A  (Stichting voor de Technische Wetenschappen),<br>02 June 2005 (02.06.2005),<br>paragraphs [0007] to [0010], [0036], [0081] to [0085]; fig. 7a<br>& US 2005/0187477 A1<br>paragraphs [0007] to [0015], [0053], [0096] to [0100]; fig. 7a<br>& GB 2402286 A          & WO 2003/063677 A1<br>& EP 1332718 A1         & DE 10392250 T | 1-5,8-12 |
| Y | JP 2015-527096 A  (Koninklijke Philips N.V.),<br>17 September 2015 (17.09.2015),<br>paragraphs [0001], [0010]<br>& US 2015/0323311 A1<br>paragraphs [0001], [0009]<br>& WO 2013/185937 A1     & EP 2861935 A1<br>& CN 104620073 A        & RU 2014145319 A | 1-5,8-12 |

Form PCT/ISA/210 (continuation of second sheet) (January 2015)

## REFERENCES CITED IN THE DESCRIPTION

**Patent documents cited in the description**

- JP 2015092151 A

**Non-patent literature cited in the description**

- **RENZHE BI et al.** *OPTICS LETTERS,* 01 May 2013, vol. 38 (9
- **F. TALBOT.** Facts relating to optical science IV. *Philos. Mag.,* vol. 9 (1836), 401-407
- **L. RAYLEIGH.** On copying diffraction-gratings, and on some phenomenon connected therewith. *Philos. Mag.,* vol. 11 (1881), 196-205